(19) Europäisches Patentamt
European Patent Office
Office européen des brevets

(11) **EP 1 834 528 B1**

(12) **EUROPEAN PATENT SPECIFICATION**

(45) Date of publication and mention of the grant of the patent:
**25.06.2014 Bulletin 2014/26**

(51) Int Cl.:
*A23L 1/05* *(2006.01)*  *A23L 1/076* *(2006.01)*
*A23L 2/385* *(2006.01)*  *A23L 2/52* *(2006.01)*
*A23L 2/60* *(2006.01)*  *A23L 1/0532* *(2006.01)*
*A61K 9/00* *(2006.01)*

(21) Application number: **04807672.3**

(22) Date of filing: **24.12.2004**

(86) International application number:
**PCT/JP2004/019315**

(87) International publication number:
**WO 2006/067850 (29.06.2006 Gazette 2006/26)**

(54) **PROCESS FOR PRODUCTION OF JELLYLIKE DRINKS**

VERFAHREN ZUR HERSTELLUNG VON GELEEARTIGEN GETRÄNKEN

PROCEDE D'ELABORATION DE BOISSONS GELIFIEES

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(43) Date of publication of application:
**19.09.2007 Bulletin 2007/38**

(73) Proprietor: **Ryukakusan Co. Ltd.**
**Tokyo 101-0031 (JP)**

(72) Inventor: **Fukui, Atsuko,**
**c/o Ryukakusan Co., Ltd.,**
**Chiyoda-ku, Tokyo 1010031 (JP)**

(74) Representative: **Gordon, Jennifer Claire**
**Dehns**
**St Bride's House**
**10 Salisbury Square**
**London EC4Y 8JD (GB)**

(56) References cited:
WO-A-2006/054886     JP-A- 2000 157 212
JP-A- 2000 191 553     JP-A- 2000 279 107
JP-A- 2003 144 064

• DATABASE WPI Week 200050 Thomson Scientific, London, GB; AN 2000-545835 XP002507522 -& JP 2000 191553 A (LION CORP) 11 July 2000 (2000-07-11)
• DATABASE WPI Week 199827 Thomson Scientific, London, GB; AN 1998-304933 XP002507523 -& JP 10 108633 A (TORIGOE SEIFUN KK) 28 April 1998 (1998-04-28)
• DATABASE BIOSIS [Online] BIOSCIENCES INFORMATION SERVICE, PHILADELPHIA, PA, US; March 2000 (2000-03), FUKUI ATSUKO ET AL: "Development of deglutition aid jelly" XP002507521 Database accession no. PREV200000236259 & YAKUZAIGAKU, vol. 60, no. 1, March 2000 (2000-03), pages 62-70, ISSN: 0372-7629

Remarks:
The file contains technical information submitted after the application was filed and not included in this specification

**Description**

The present invention relates to a process for producing a jellylike drink, more particularly to a process for producing a jellylike drink which is a swallowing-assistive drink facilitating swallowing of a medicine.

Medicines have been conventionally taken together with water or warm water in general. However, it is difficult for patients of swallowing difficulties, particularly, for elderly people, to take medicines with such water or warm water, and when they take medicines in the form of powders, granules, capsules, tablets and the like, they cannot swallow them to cause choking or remaining of medicines in the mouth. Consequently, satisfactory remedial effects are not obtained or the patients themselves sometimes feel displeasure.

In connection therewith, an attempt to use jellylike drinks as swallowing-assistive drinks has been made paying an attention to their features of smooth passing through throat. For example see JP 2000-191553, JP 1998-108663 and WO 2006/054886. The present applicant has already proposed a low-calorie non-sugar swallowing-assistive drink containing a paste material, such as agar or carrageenan, and mannitol and having a specific jelly strength (see Japanese patent document other gelling agents and a sweetering agent no.3257983).

Because such a swallowing-assistive drink is used also by a person of swallowing difficulties, safety is regarded as important, and for the safety, it is desirable to stably keep physical property values such as jelly strength and water release ratio.

By the way, a jellylike drink used as such a swallowing-assistive drink is produced by dissolving a gelling agent in hot water or the like having a temperature exceeding 80°C and then mixing the resulting solution with components other than the gelling agent, such as a sweetening agent, similarly to usual jelly products. The reason is that it has been believed that formation of insoluble lumps is inhibited and the jelly strength is easily kept constant by previously dissolving the gelling agent in hot water. See, for example JP 2000-279107, which discloses a powdery mixture comprising gelatin as a main gelling agent and one or more.

In order to carry out the process, however, the apparatus such as a mixing tank must be maintained at a temperature exceeding 80°C (e.g., 90 to 100°C), and special facilities for the maintenance are necessary. Further, there is another problem that the high temperature-maintaining time is required.

In fact, as the temperature of hot water to dissolve the gelling agent becomes higher, and as the time to maintain the system at the temperature is prolonged, the amount of heat energy given to the gelling agent becomes larger to increase a hydrolysis reaction rate of the gelling agent and thereby promote decomposition of the gelling agent. As a result, the water release ratio of the resulting jellylike product, particularly the water release ratio thereof after the lapse of time, is increased, and control of physical property values such as jelly strength becomes difficult. Moreover, even if such a high temperature is used, the gelling agent is aggregated in hot water to form insoluble lumps, so that a step of removing the lumps must be incorporated into the production process, and this hinders simplification of the production process.

Such a problem is conspicuously observed particularly when agar is used as the gelling agent.

In the light of such circumstances as mentioned above, the present inventor has earnestly studied, and as a result, she has found that the gelling agent can be dissolved even in water at not higher than 80°C by previously mixing the gelling agent with a sweetening agent in a powdery state. Based on the finding, the present invention has been accomplished.

That is to say, it is an object of the present invention to provide a process for producing a jellylike drink, which has a step of dissolving both of a gelling agent and a sweetening agent in water at not higher than 80°C by the use of a mixed powder obtained by previously mixing the gelling agent with the sweetening agent in a powdery state.

More particularly, it is an object of the present invention to provide a process for producing a jellylike drink, which solves the problems of the prior art and in which special production facilities for maintaining the system at a high temperature exceeding 80°C are not required, simplification of the production process and shortening of the production time are promoted, and a jellylike drink having stable physical property values is produced with high production efficiency.

The process for producing a jellylike drink according to the present invention comprises a mixing step of mixing a powdery gelling agent with a powdery sweetening agent (I) to obtain a mixed powder and a dissolving step (I) of dissolving the resulting mixed powder in water in the range of 60 to 70°C, wherein the powdery gelling agent is agar or a mixture of agar and at least one selected from carrageenan, gellan gum, peckin, locust bean gum, tara gum, guar gum, xanthan gum, mannan, tamarind gum, starch and modified starch; the sweetening agent (I) is used in an amount of not less than 50 parts by weight based on 100 parts by weight of the gelling agent in the mixing step; and stirring is carried out with maintaining the temperature of the system after addition of the mixed powder at 60 to 70°C in the dissolving step (I).

The present invention may further comprise a dissolving step (II) of dissolving a sweetening agent (II) in a solution obtained in the dissolving step (I).

The jellylike drink produced by the present invention is preferably a swallowing-assistive drink which is taken together with a medicine and, facilitates swallowing of the medicine. The jelly strength of the swallowing-assistive drink at 20°C is preferably in the range of 10 to 100 g/cm$^2$.

According to the process for producing a jellylike drink of the present invention, the gelling agent can be dissolved in water in the range of 60 to 70°C together with a sweetening agent, and special production facilities for maintaining the system at a high temperature exceeding 80°C are unnecessary. Moreover, lumps are not formed and the production process can be simplified, so that the production time can be shortened, and enhancement of production efficiency is feasible.

The jellylike drink obtained by the present invention is not maintained at a high temperature exceeding 80°C when the gelling agent is dissolved, and therefore, decomposition reaction of the gelling agent is inhibited. In the case where the jellylike drink is used as, for example, a swallowing-assistive drink, the water release ratio after the lapse of time is low, and stable appearance and performance of the product can be kept over a long period of time.

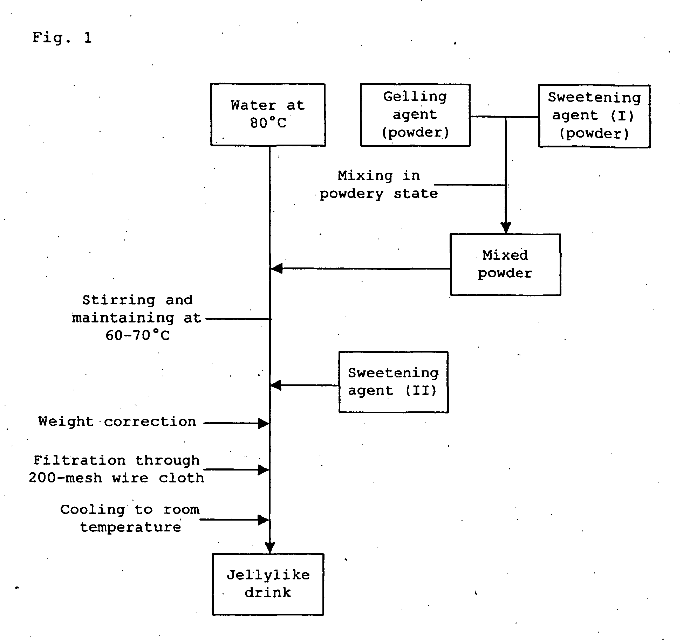

Fig. 1 is a schematic flow chart of Example 1.

Hereafter, the present invention is described specifically.

As described above, the process for producing a jellylike drink of the invention comprises a mixing step of mixing a powdery gelling agent with a powdery sweetening agent (I) to obtain a mixed powder and a dissolving step (I) of dissolving the resulting mixed powder in water in the range of 60 to 70°C.

More specifically, in the mixing step, the gelling agent and a sweetening agent (I) are previously mixed in a powdery state.

In the conventional process for producing a jelly article such as a jellylike drink, it is intended to inhibit formation of insoluble lumps by previously dissolving a gelling agent in hot water exceeding 80°C. In fact, however, formation of insoluble lumps cannot be prevented even if such a treatment is carried out. The reason is presumably that the gelling agent is liable to be aggregated in water because the gelling agent has a small surface area, and after the gelling agent is once aggregated, only the surface of the aggregate is swollen but the inside thereof is not swollen.

A method of introducing a small amount of a gelling agent into hot water through a sieve or the like over a long period of time has been studied, but similarly to the above case, formation of lumps cannot be inhibited.

Further, even if the temperature of hot water is raised in order to dissolve the gelling agent rapidly or the high temperature-maintaining time is prolonged, formation of insoluble lumps cannot be inhibited. On the contrary, there arises a vicious circle that hydrolysis reaction of the gelling agent is accelerated to induce decomposition of the gelling agent itself and this leads to deterioration of appearance or performance of the resulting jelly product, particularly deterioration of appearance or performance thereof after the lapse of time, such as water release.

In contrast therewith, the present invention has the aforesaid mixing step. In the mixing step, the gelling agent and the sweetening agent (I) are previously mixed in a powdery state, whereby the gelling agent is surrounded with the sweetening agent (I) to increase an apparent surface area of the gelling agent. Further, by allowing particles of the sweetening agent (I) to be present among the individual particles of the gelling agent, an effect that the gelling agent particles are prevented from direct contact with one another to prevent aggregation of the gelling agent in water is expected.

Accordingly, it is thought that the individual particles of the gelling agent are in a sufficiently dispersed state even in water at not higher than 80°C used in the subsequent dissolving step (I), immediately after they are mixed with the water, and even after the sweetening agent (I) is rapidly dissolved in the water, the sufficiently dispersed state lasts, and the individual gelling agent particles in the sufficiently dispersed state are swollen with water not only on the surfaces thereof but also inside thereof and are dissolved without formation of insoluble lumps due to aggregation of the gelling agent particles.

That is to say, by the use of a mixed powder of the gelling agent and the sweetening agent (I), it becomes feasible to dissolve the gelling agent together with the sweetening agent (I) in water in the range of 60 to 70° C.

As the gelling agent employable in the invention, publicly known gelling agents which are powdery at room temperature and suitable for eating and drinking can be used without any restriction. Examples of such gelling agents include agar, carrageenan, gellan gum, furcelleran, gelatin, pectin, curdlan, locust bean gum, tara gum, guar gum, xanthan gum, alginic acid, alginate, azotobacter vinelandii gum, cassia gum, psyllium seed gum, mannan, tamarind gum, carboxymethyl cellulose salt, milk serum protein, starch and modified starch. These gelling agents can be used singly or as a mixture of two or more kinds.

Of the above gelling agents, preferable is agar, carrageenan, gellan gum, pectin, locust bean gum, tara gum, guar gum, xanthan gum, mannan, tamarind gum, starch or modified starch, or a mixture of agar and at least one component selected from these gelling agents except agar. More preferable is agar, or a mixture of agar and at least one component selected from these gelling agents except agar. And, still more preferable is agar, from the viewpoint that the effect of the invention is preferably exerted.

The gelling agent is used so as to be contained in an amount of usually 0.1 to 5.0% by weight in the whole amount of the jellylike drink.

As the sweetening agent (I) employable in the invention, any of publicly known sweetening agents which are powdery at room temperature can be used without any restriction. Examples of such sweetening agents include sugar alcohols such as reducing maltose, reducing maltose thick malt syrup, reducing thick malt syrup, reducing lactose, xlytol, erythritol, sorbitol, mannitol, maltitol, lactitose and palatinose; saccharides such as sucrose, maltose, maltose thick malt syrup, lactose, fructose, glucose, oligosaccharide, dextrin, trehalose, honey and starch; and stevia, sucralose, Luo Han Guo (*Momordica grosvenori*), aspartame and saccharin. These sweetening agents may be used singly or as a mixture of two or more kinds. In the case where it is taken into account that the resulting drink is used as a swallowing-assistive drink by a diabetic or in the case where the resulting drink is used as a low-calorie non-sugar drink, preferable are sugar alcohols of the above sweetening agents, and of these, more preferable is erythritol.

In the mixing step, the sweetening agent (I) is used in an amount of not less than 50 parts by weight, preferably not less than 100 parts by weight, based on 100 parts by weight of the gelling agent. The upper limit of the amount of the sweetening agent (I) used is desirably not more than 2400 parts by weight based on 100 parts by weight of the gelling agent.

By the use of the sweetening agent (I) in the above amount based on the gelling agent, the gelling agent is surrounded with the sweetening agent (I) to moderately increase an apparent surface area of the gelling agent when they are mixed in a powdery state, and besides, the sweetening agent (I) is allowed to be present among the gelling agent particles to prevent direct contact of the gelling agent particles with one another and thereby prevent aggregation of the gelling agent in water.

The means for mixing the gelling agent with the sweetening agent (I) is not specifically restricted, and a powder mixing means publicly known can be properly adopted. For example, mixing using a V-type mixing machine, a stirring mixing machine, a mixer or the like is employable. The mixing time is not specifically restricted provided that they are sufficiently mixed, though it varies depending upon the mixing means. For example, when a stirring mixing machine is used, the mixing time is about 20 seconds.

Next, the mixed powder obtained in the above mixing step is dispersed and dissolved in water at not higher than 80°C in the dissolving step (I).

In the mixed powder obtained in the mixing step, the gelling agent is surrounded with the sweetening agent (I) to increase an apparent surface area of the gelling agent, and the sweetening agent (I) is allowed to be present among the gelling agent particles to prevent direct contact of the gelling agent particles with one another. Therefore, even in water in the range of 60 to 70°C, individual particles of the gelling agent, which are in a sufficiently dispersed state, are swollen with water even inside the particles and are dissolved without formation of insoluble lumps due to aggregation of the gelling agent particles.

The water employable in the invention has only to be water that is suitable for drinking, and examples thereof include distilled water, natural spring water, tap water, ion-exchanged water and purified water.

The water has only to be used in such an amount that the total amount of water and the above-described or later-described components of the jellylike drink other than water becomes 100% by weight, that is, the water has only to be used so as to be contained as residues. The water may be added in the whole amount in the dissolving step (I), or the water may be added partially in the dissolvent step (I) and added in the residual amount in any of the later steps.

The temperature of the water used in the dissolving step (I) is in the range 60 to 70°C.

After the mixed powder is added, stirring of the whole system is carried out, when needed, to dissolve the mixed powder in water in the range of 60 to 70°C, and in this case, stirring is desirably carried out with maintaining the temperature of the system after addition of the mixed powder, at 60 to 70°C. By combining the temperature control with the stirring operation, dissolution of the mixed powder is carried out more rapidly.

It is also possible that the mixed powder is introduced into water at 40 to 60°C, stirred and dispersed, and then the temperature of the water is raised to 70 to 80°C to dissolve the mixed powder. In this case, however, facilities capable of raising the temperature become necessary.

In the present invention, the mixed powder containing the gelling agent is dissolved in water in the range of 60 to 70°C as described above. Therefore, special facilities for maintaining the system at a high temperature exceeding 80°C and the high temperature-maintaining time are unnecessary in the dissolving step. Moreover, decomposition of the gelling agent is inhibited, and therefore, appearance and performance of the resulting jellylike drink can be kept stably over a long period of time.

In the present invention, a dissolving step (II) in which a sweetening agent (II) is further dissolved in a solution obtained in the aforesaid dissolving step (I) may be included. By virtue of the dissolving step (II), control of taste or jelly strength of the jellylike drink becomes feasible. Also in this case, it is preferable to perform stirring with maintaining the temperature of the system after addition of the sweetening agent (II), at 60 to 70°C, from the viewpoint of rapid dissolution.

Examples of the sweetening agents (II) employable in the invention include the same ones as previously described for the sweetening agent (I). In addition, sweetening agents which are liquid at room temperature, such as single syrup, honey, thick malt syrup, reducing maltose thick malt syrup, maple syrup, lactitol and palatinose, are also employable. The sweetening agent (II) can be used in such an amount that the sweetening agent (I) and the sweetening agent (II) are contained in the total amount of usually 3.0 to 25.0% by weight in the whole amount of the jellylike drink.

In the present invention, a step of adding various additives, e.g., pH adjustors, such as citric acid and sodium citrate, fats and oils of animals and plants, surface-active agents, antiseptics, flavorings, coloring matters and vitamins, in amounts not detrimental to the effects of the invention, when needed, may be further included.

In the case where the whole amount of water is not used in the dissolving step (I), the residual amount of water may be added in the dissolving step (II) or in the step of adding various additives, or a step of adding only the residual amount of water may be included.

Thereafter, the system is cooled to room temperature, whereby a jellylike drink can be obtained.

According to the process of the invention, formation of insoluble lumps can be prevented, and therefore, it is not essential to provide a step of removing lumps such as a filtration step prior to the cooling step. However, the process of the invention does not exclude an embodiment having such a step.

Further, a sterilization step of maintaining the resulting jellylike drink at a temperature exceeding 80°C, such as 90 to 100°C, for 30 seconds to 3 minutes may be provided, when needed. Such a short period of time exerts little influence on the gelling agent, and there is scarcely any fear of decomposition of the gelling agent.

The jellylike drink obtained by the production process of the invention can be preferably used as a swallowing-assistive drink which is taken together with a medicine and facilitates swallowing of the medicine. In the case where the jellylike drink is used as such a swallowing-assistive drink, the jelly strength of the jellylike drink at 20°C is in the range of usually 10 to 100 $g/cm^2$, preferably 10 to 60 $g/cm^2$. When the jelly,strength is in the above range, there is no possibility of occurrence of troubles such as choking even in case of a dysphagia person, and smooth swallowing becomes feasible. Needless to say, the jellylike drink desirably has a jelly strength of the above range even after the lapse of time.

For the convenience of usage and portage, it is preferable to pack the jellylike drink and the swallowing-assistive drink in containers. The container is, for example, an aluminum pack with a spout.

The present invention is further described with reference to the following examples, but it should be construed that the invention is in no way limited to those examples.

Example 1

Preparation of jellylike drink (E1)

In a V-type mixing machine (50 liters), 20 kg of powdery erythritol and 13.8 kg of agar were mixed for 2 minutes to obtain a mixed powder.

In a 1000-liter tank having a stirring blade, 820.85 kg of ion-exchanged water at 80°C was placed, and with rotating the stirring blade at 50 rpm, the mixed powder obtained above was added. Then, with maintaining the tank at $65\pm5$°C, stirring was carried out for 10 minutes to dissolve the mixed powder.

With maintaining the tank at $65\pm5$°C, 60 kg of erythritol was further added to the tank, and stirring was carried out for 5 minutes to dissolve the erythritol. Thereafter, 0.35 kg of stevia and 85 kg of sorbitol were added, then stirring was carried out for 5 minutes to dissolve them, and weight correction with water was made so that the weight should become 1000 kg.

Thereafter, stirring was further carried out for another 1 minute. Then, in order to examine presence of insoluble lumps, the resulting mixture was filtered through a 200-mesh wire cloth and cooled to room temperature to obtain a jellylike drink (E1).

In Fig. 1, a schematic flow chart of the process for producing the jellylike drink (E1) of Example 1 is shown.

Evaluation of jellylike drink (E1)

The resulting jellylike drink (E1) was evaluated on (1) jelly strength, (2) presence of insoluble lumps, (3) water release ratio after the lapse of time and (4) jelly strength after the lapse of time in accordance with the following methods and evaluation criteria. The results are set forth in Table 1.

(1) Jelly strength

Jelly strength of the jellylike drink (E1) was measured by means of a rheometer (manufactured by Sun Scientific Co., Ltd., Rheometer MODEL COMPAC-100) under the conditions of a plunger diameter of 2'cm, a compression rate of 10 mm/sec and a measuring temperature of 20°C.

(2) Presence of insoluble lumps

The jellylike drink (E1) in the state (65±5°C) previous to cooling of the jellylike drink to room temperature was allowed to pass through a 200-mesh wire cloth, and the amount of a solid matter which had not passed through the wire cloth was visually observed, and presence of insoluble lumps was evaluated based on the following criteria.

AA: A solid matter remaining on the wire cloth is not observed.

BB: A small amount of a solid matter remains on the wire cloth.

CC: A large amount of a solid matter remains on the wire cloth.

(3) Water release ratio after the lapse of time

In a closed container charged with nitrogen, 200 g of the jellylike drink (E1) was placed and allowed to stand still for 1 month at 40°C, and then the amount of water released from the jellylike drink was measured at room temperature. From the formula:

$$\text{water release ratio (\%)} = 100$$
$$\times \text{ amount of water released (g)/water content of jellylike}$$
$$\text{drink } (200 \times 820.85/1000)\text{(g)},$$

a water release ratio(%) was calculated, followed by evaluation based on the following criteria.

AA: The water release ratio is less than 5%.
BB: The water release ratio is not less than 5% and less than 10%.
CC: The water release ratio is not less than 10%.

(4) Jelly strength after the lapse of time

In a closed container charged with nitrogen, the jellylike drink (E1) was placed and allowed to stand still for 1 month at 40°C, and then a jelly strength of the jellylike drink (E1) was measured by means of a rheometer (manufactured by Sun Scientific Co., Ltd., Rheometer MODEL COMPAC-100) under the conditions of a plunger diameter of 2 cm, a compression rate of 10 mm/sec and a measuring temperature of 20°C.

Example 2

Preparation of jellylike drink (E2)

A jellylike drink (E2) was obtained in the same manner as in Example 1, except that the temperature of the ion-exchanged water used first was changed to 70°C from 80°C.

Evaluation of jellylike drink (E2)

The resulting jellylike drink (E2) was evaluated on (1) jelly strength, (2) presence of insoluble lumps, (3) water release ratio after the lapse of time and (4) jelly strength after the lapse of time in the same manner as in Example 1. The results are set forth in Table 1.

Comparative Example 1

Preparation of jellylike drink (C1)

In a 1000-liter tank having a stirring blade, 820.85 kg of ion-exchanged water at 90°C was placed, and with rotating the stirring blade at 50 rpm, 13.8 kg of powdery agar was added. Then, with maintaining the tank at 90±5°C, stirring was carried out to dissolve the agar seemingly. It took 30 minutes to seemingly dissolve the agar.

With maintaining the tank at 90±5°C, 80 kg of erythritol was further added, and stirring was carried out for 5 minutes to dissolve the erythritol. Thereafter, 0.35 kg of stevia and 85 kg of sorbitol were added, then stirring was carried out for 5 minutes to dissolve them, and weight correction with water was made so that the weight should become 1000 kg. Thereafter, stirring was further carried out for another 1 minute. Then, in order to examine presence of insoluble lumps, the resulting mixture was filtered through a 200-mesh wire cloth and cooled to room temperature to obtain a jellylike drink (C1).

Evaluation of jellylike drink (C1)

The resulting jellylike drink (C1) was evaluated on (1) jelly strength, (2) presence of insoluble lumps, (3) water release ratio after the lapse of time and (4) jelly strength after the lapse of time in the same manner as in Example 1. The results are set forth in Table 1.

Comparative Example 2

Preparation of jellylike drink (C2)

In a 1000-liter tank having a stirring blade, 820.85 kg of ion-exchanged water at 80°C was placed, and then with rotating the stirring blade at 50 rpm, 13.8 kg of powdery agar was added. Then, with maintaining the tank at $65\pm5$°C, stirring was carried out for 10 minutes.
With maintaining the tank at $65\pm5$°C, 80 kg of erythritol was further added, and stirring was carried out for 5 minutes. Thereafter, 0.35 kg of stevia and 85 kg of sorbitol were added, then stirring was carried out for 5 minutes, and weight correction with water was made so that the weight should become 1000 kg.
Thereafter, stirring was further carried out for another 1 minute. Then, in order to examine presence of insoluble lumps, the resulting mixture was filtered through a 200-mesh wire cloth and cooled to room temperature to obtain a jellylike drink (C2).

Evaluation of jellylike drink (C2)

The resulting jellylike drink (C2) was evaluated on (1), jelly strength, (2) presence of insoluble lumps, (3) water release ratio after the lapse of time and (4) jelly strength after the lapse of time in the same manner as in Example 1. The results are set forth in Table 1.

Comparative Example 3

Preparation of jellylike drink (C3)

A jellylike drink (C3) was obtained in the same manner as in Example 1, except that the temperature of the ion-exchanged water used first was changed to 90°C from 80°C and the temperature of the tank after that was maintained at $90\pm5$°C.

Evaluation of jellylike drink (C3)

The resulting jellylike drink (C3) was evaluated on (1) jelly strength, (2) presence of insoluble lumps, (3) water release ratio after the lapse of time and (4) jelly strength after the lapse of time in the same manner as in Example 1. The results are set forth in Table 1.
Table 1

| | Jellylike drink | (1) Jelly strength (g/cm$^2$) | (2) Presence of lumps | (3) Water release ratio after the lapse of time | (4) Jelly strength after the lapse of time (g/cm$^2$) |
|---|---|---|---|---|---|
| Ex. 1 | E1 | 48 | AA | AA | 50 |
| Ex. 2 | E2 | 42 | AA | AA | 43 |
| Comp. Ex. 1 | C1 | 25 | BB | CC | 34 |
| Comp. Ex. 2 | C2 | 8 | CC | BB | 13 |

(continued)

|  | Jellylike drink | (1) Jelly strength (g/cm$^2$) | (2) Presence of lumps | (3) Water release ratio after the lapse of time | (4) Jelly strength after the lapse of time (g/cm$^2$) |
|---|---|---|---|---|---|
| Comp. Ex. 3 | C3 | 53 | AA | CC | 62 |

It can be seen from Table 1 that in Example 1 and Example 2 in which the gelling agent and the sweetening agent were previously mixed, the resulting mixed powder was added to water at 80°C or 70°C and the system was maintained at 65±5°C, formation of insoluble lumps was not observed, and the water release ratio after the lapse of time was low. It can be also seen that the jellylike drinks obtained by these examples kept a preferred jelly strength for swallowing-assistive drinks not only immediately after preparation but also after the lapse of time.

On the other hand, in Comparative Example 1 (conventional process for producing jelly articles) in which only the gelling agent was added to hot water at 90°C and the system was maintained at 90±5°C, formation of insoluble lumps was observed though the jelly strength was within the preferred range for swallowing-assistive drinks. Further, the water release ratio after the lapse of time was high, and the jelly strength after the lapse of time was also high. From these facts, keeping of stable physical property values over a long period of time has proved to be difficult.

In Comparative Example 2 in which only the gelling agent was added to water at 80°C without previously mixing the gelling agent with the sweetening agent and the system was maintained at 65±5°C, aggregation of the gelling agent was conspicuous and large amounts of insoluble lumps were formed. Further, the jelly strength immediately after preparation was out of a preferred range for swallowing-assistive drinks. Moreover, the water release ratio after the lapse of time was high, and the jelly strength after the lapse of time also varied. From these facts, keeping of stable physical property values over a long period of time has proved to be difficult.

In Comparative Example 3 in which the gelling agent and the sweetening agent were previously mixed, the resulting mixed powder was added to water at 90°C and the system was maintained at 90±5°C, the water release ratio after the lapse of time was high though the jelly strength immediately after preparation was within a preferred range for swallowing-assistive drinks. Further, the jelly strength after the lapse of time also varied. From these facts, keeping of stable physical property values over a long period of time has proved to be difficult.

<u>INDUSTRIAL APPLICABILITY</u>

According to the present invention, special production facilities for maintaining the system at a high temperature exceeding 80°C and the high temperature-maintaining time are unnecessary when the gelling agent is dissolved in the production of a jellylike drink, as described above. In addition, lumps are not formed, and the production process can be simplified, so that the production time can be shortened and enhancement of production efficiency is feasible.

The jellylike drink obtained by the invention is not maintained at a high temperature exceeding 80°C when the gelling agent is dissolved, and therefore, decomposition reaction of the gelling agent is inhibited. For example, in the case where the jellylike drink is used as a swallowing-assistive drink, the water release ratio after the lapse of time is low, and stable appearance and performance of the product can be kept over a long period of time.

Accordingly, the process for producing a jellylike drink of the invention is useful as a process for producing all the jellylike drinks, particularly swallowing-assistive drinks, and is useful for the industry of manufacturing the articles.

**Claims**

1. A process for producing a swallowing-assistive jellylike drink facilitating swallowing of a medicine, comprising a mixing step of mixing a powdery gelling agent with a powdery sweetening agent (I) to obtain a mixed powder and a dissolving step (I) of dissolving the resulting mixed powder in water in the range of 60 to 70°C, wherein the powdery gelling agent is agar or a mixture of agar and at least one selected from carrageenan, gellan gum, pectin, locust bean gum, tara gum, guar gum, xanthan gum, mannan, tamarind gum, starch and modified starch; in the mixing step, the sweetening agent (I) is used in an amount of not less than 50 parts by weight based on 100 parts by weight of the gelling agent; and in the dissolving step (I), stirring is carried out with maintaining the temperature of the system after addition of the mixed powder at 60 to 70°C.

2. The process for producing a jellylike drink as claimed in claim 1, further comprising a dissolving step (II) of dissolving a sweetening agent (II) in a solution obtained in the dissolving step (I).

3. The process for producing a jellylike drink as claimed in claim 1 or 2, wherein the jellylike drink is a swallowing-assistive drink which is taken together with a medicine and facilitates swallowing of the medicine.

4. The process for producing a jellylike drink as claimed in claim 3, wherein the jelly strength of the swallowing-assistive drink at 20°C is in the range of 10 to 100 g/cm$^2$.

**Patentansprüche**

1. Verfahren zur Herstellung eines das Schlucken unterstützenden geleeartigen Getränks, welches das Schlucken einer Medizin vereinfacht, umfassend einen Schritt des Mischens eines pulverförmigen Geliermittels mit einem pulverförmigen Süßungsmittel (I), um ein gemischtes Pulver zu erhalten, sowie einen Schritt des Auflösens (I), bei dem das resultierende gemischte Pulver in Wasser im Bereich von 60 bis 70°C aufgelöst wird, wobei das pulverförmige Geliermittel Agar ist, oder eine Mischung aus Agar und wengistens einem ausgewählt aus Carrageen, Gellan, Pektin, Johannisbrotkernmehl, Tarakernmehl, Guarkernmehl, Xanthan, Mannan, Tamarindkernmehl, Stärke und modifizierte Stärke;
beim Schritt des Mischens wird das Süßungsmittel (I) in einer Menge von nicht weniger als 50 Gewichtsteilen basierend auf 100 Gewichtsteilen des Geliermittels verwendet; und beim Schritt des Auflösens (I) wird das Rühren unter einer gleichbleibenden Temperatur des Systems nach dem Hinzufügen des gemischten Pulvers bei 60 bis 70°C durchgeführt.

2. Verfahren zur Herstellung eines geleeartigen Getränks nach Anspruch 1, ferner umfassend einen Schritt des Auflösens (II) zum Auflösen eines Süßungsmittels (II) in einer Lösung, die im Auflösungsschritt (I) erhalten wird.

3. Verfahren zur Herstellung eines geleeartigen Getränks nach Anspruch 1 oder 2, wobei das geleeartige Getränk ein Getränk ist, welches das Schlucken unterstützt, das zusammen mit einer Medizin eingenommen wird, und das Schlucken der Medizin vereinfacht.

4. Verfahren zur Herstellung eines geleeartigen Getränks nach Anspruch 3, wobei die Geleestärke des Getränks, welches das Schlucken unterstützt, bei 20°C im Bereich von 10 bis 100 g/cm$^2$ liegt.

**Revendications**

1. Processus pour produire une boisson gélatineuse d'aide à la déglutition facilitant la déglutition d'un médicament, comprenant une étape de mélange consistant à mélanger un agent gélifiant pulvérulent avec un agent édulcorant pulvérulent (I) pour obtenir une poudre mélangée et une étape de dissolution (I) consistant à dissoudre la poudre mélangée résultante dans de l'eau dans la plage de 60 à 70 °C, dans lequel l'agent gélifiant pulvérulent est de la gélose ou un mélange de gélose et d'au moins un élément sélectionné parmi la carraghénine, la gomme de gellane, la pectine, la gomme de caroube, la gomme de tara, la farine de guar, la gomme de xanthane, le mannane, la gomme de tamarin, l'amidon et l'amidon modifié ; dans l'étape de mélange, l'agent édulcorant (I) est utilisé en une quantité non inférieure à 50 parties en poids sur la base de 100 parties en poids de l'agent gélifiant ; et dans l'étape de dissolution (I), une agitation est effectuée tout en maintenant la température du système après ajout de la poudre mélangée à 60 à 70 °C.

2. Processus pour produire une boisson gélatineuse selon la revendication 1, comprenant en outre une étape de dissolution (II) consistant à dissoudre un agent édulcorant (II) dans une solution obtenue dans l'étape de dissolution (I).

3. Processus pour produire une boisson gélatineuse selon la revendication 1 ou 2, dans lequel la boisson gélatineuse est une boisson d'aide à la déglutition qui est prise en même temps qu'un médicament et qui facilite la déglutition du médicament.

4. Processus pour produire une boisson gélatineuse selon la revendication 3, dans lequel la consistance de la gelée de la boisson d'aide à la déglutition à 20 °C est dans la plage de 10 à 100 g/cm$^2$.

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000191553 A
- JP 10108663 A
- WO 2006054886 A
- JP 3257983 B
- JP 2000279107 A